# EUROPEAN PATENT APPLICATION

(11) **EP 4 032 970 A1**
(43) Date of publication of application: **27.07.2022**
(21) Application number: 20865363.4
(22) Date of filing: 15.09.2020
(51) Int. Cl.: C12N 5/073, A61K 35/32, A61K 47/36, A61K 9/00, A61P 19/00

(54) **STEM CELLS DERIVED FROM VILLI ADJACENT TO CHORIONIC PLATE, AND TISSUE REGENERATION CELL THERAPEUTIC AGENT COMPRISING SAME**

(30) Priority: 19.09.2019 KR 20190115322
(71) Applicant: Ha, Yoo Jin, Jeju-do 63046 (KR)
(72) Inventor: Ha, Yoo Jin, Jeju-do 63046 (KR)
(74) Representative: Cabinet Becker et Associés
(86) International application number: PCT/KR2020/012417
(87) International publication number: WO 2021/054692

(57) **Abstract**

The present invention relates to stem cells derived from villi adjacent to the chorionic plate (VCP), and a cell therapeutic agent comprising same. Stem cells derived from the tissue of VCP comprising, of the total villi, the region that is 1/3 of the distance from the area adjacent to the chorionic plate up to the villus distal part, and the basal region of the villi, according to the present invention, exhibit uniform growth characteristics, and proliferation characteristics superior to those of stem cells derived from other placental tissue, and exhibit remarkably excellent differentiation into cartilage, bone and fat, thereby being effectively usable in various tissue regeneration treatments requiring the regeneration of cartilage, bone and fat, and, particularly, in cartilage regeneration and osteoarthritis treatment.

## Description

### [Technical Field]

The present invention relates to a stem cell derived from villi adjacent to chorionic plate and cellular therapeutic agent including the same.

### [Background Art]

Stem cells refer to cells that have the ability to differentiate into two or more cells while having the ability to self-replicate, and they can be classified as totipotent stem cells, pluripotent stem cells, and multipotent stem cells. The totipotent stem cells are cells with the property of being pluripotent that can develop as a complete individual. Cells have these properties up to the 8th cell stage after fertilization of an egg and sperm. If these cells are isolated and implanted in the uterus, they can develop into a complete individual. The pluripotent stem cells are cells that can develop into various cells and tissues derived from ectoderm, mesoderm, and endoderm. They are derived from the inner cell mass which is located inside the blastocyst that appears 4-5 days after fertilization. These are called embryonic stem cells, and they differentiate into various other tissue cells, but do not form new life. Multipotent stem cells are stem cells that can differentiate only into cells specific to the tissues and organs that contain these cells. As sources of multipotent stem cells as described above, adult bone marrow, skin, blood vessels, muscles, and the like are known. These stem cells are rapidly being applied to tissue engineering, gene therapy, and cell therapy. In addition, the stem cells are obtained from various tissues, and various applications thereof are urgently required.

The placenta is an organ that develops from the uterine wall when a woman is pregnant, has a disk-like shape and is rich in vascular tissue. The fetal nutrition, respiration, and excretion are all carried out through the placenta. Scientific research results are reported that lifelong health is determined during the fetal period. Thus, the importance of the placenta during pregnancy is being emphasized, and many efforts are being made to investigate the interrelationship of various substances related thereto. The placenta is composed of various types of cells by age and location, but its application is still very limited.

Recently, studies are also being conducted on stem cells isolated from placental tissue. Most of the conventional placental tissue-derived stem cells are stem cells derived from some tissues of the term placenta obtained during childbirth. However, the placenta is composed of various tissues, such as the basement membrane attached to the uterus, the chorionic tissue that mediates the exchange of substances between mother and fetal blood as the central tissue of the placenta, and the chorionic membrane that covers the placenta inside the placenta and surrounds the fetus and the amniotic membrane. Until now, studies on these sub-organisms were not widely known. Meanwhile, Korean Patent Registration No. 818214 discloses a method for isolating stem cells from the amniotic membrane or decidua using N-acetyl-L-cysteine NAC)-containing medium, and Korean Patent Registration No. 871984 discloses the multipotency of stem cells derived from amniotic membrane, serous membrane, basal decidua and placental tissues using basic fibroblast growth factor (bFGF)-containing medium.

However, regardless of the method for distinguishing the placental structure anatomically known in the prior art, it has not yet been widely reported on a method for subdividing the placenta or stem cells obtained from the segmented region and their effects.

### [Disclosure]

### [Technical Problem]

Therefore, the present inventors define a new detailed site of the placenta regardless of the conventional method for distinguishing placental tissue. Accordingly, the present inventors completed the present invention by obtaining a villi adjacent to chorionic plate (VCP) while studying the stem cell characteristics and confirming that the isolated stem cells from VCP had different CD marker expression patterns and significantly excellent ability to differentiate into cartilage, bone and fat compared to villi-derived stem cells from the rest except for the villi adjacent to the chorionic plate of whole villi, whole placenta and whole villi.

Therefore, an object of the present invention is to provide stem cells derived from villi adjacent to chorionic plate (VCP) tissue. The villi adjacent to the chorionic plate is the villi of the region from the region adjacent to the chorionic plate to a 1/3 point of the whole thickness of the region from adjacent to the chorionic plate to the end of the villi, and includes the basal portion of the villi. Further, another object of the present invention is to provide a cellular therapeutic agent, a composition for tissue regeneration, and a method for separating them using stem cells derived from the villi adjacent to chorionic plate tissue.

### [Technical Solution]

In order to achieve the above object, the present invention provides a stem cell derived from villi adjacent to chorionic plate (VCP) tissue, in which the villi adjacent to chorionic plate is the villi of the region from the region adjacent to the chorionic plate to a 1/3 point of the whole thickness of the region from adjacent to the chorionic plate to the end of the villi, and includes the basal portion of the villi.

Further, the present invention provides a method for separating and obtaining a stem cell derived from villi adjacent to chorionic plate (VCP), the method including: 1) obtaining villi adjacent to chorionic plate (VCP) tissue by cutting a portion from the region adjacent to the chorionic plate to a 1/3 point of the whole thickness of the region from adjacent to the chorionic plate to the end of the villi, and includes the basal portion of the villi; and 2) obtaining a stem cell from the villi adjacent to the chorionic plate.

Further, the present invention provides a method for preparing a stem cell derived from villi adjacent to chorionic plate (VCP), the method including: 1) obtaining villi adjacent to chorionic plate (VCP) by cutting a portion from the region adjacent to the chorionic plate to a 1/3 point of the whole thickness of the region from adjacent to the chorionic plate to the end of the villi, and includes the basal portion of the villi; and 2) obtaining a stem cell from the villi adjacent to the chorionic plate.

Further, the present invention provides a cellular therapeutic agent for tissue regeneration including the stem cell as an active ingredient.

Further, the present invention provides a composition for tissue regeneration including the stem cell as an active ingredient.

Further, the present invention provides a method for tissue regeneration, the method including a step of: treating an individual in need of a stem cells, in which the stem cells are derived from villi adjacent to chorionic plate(VCP) tissue, in which the villi adjacent to chorionic plate is the villi of the region from the region adjacent to the chorionic plate to a 1/3 point of the whole thickness of the region from adjacent to the chorionic plate to the end of the villi, and includes the basal portion of the villi.

Further, the present invention provides a method for tissue generation, the method including steps of: 1) obtaining villi adjacent to chorionic plate (VCP) by cutting a portion from the region adjacent to the chorionic plate to a 1/3 point of the whole thickness of the region from adjacent to the chorionic plate to the end of the villi, and includes the basal portion of the villi; 2) obtaining stem cells from the villi adjacent to the chorionic plate; and 3) treating an individual in need of the obtained stem cells derived from villi adjacent to chorionic plate.

### [Advantageous Effects]

The stem cell derived from villi adjacent to chorionic plate (VCP) tissue of the present invention is characterized in that the villi adjacent to the chorionic plate is the villi of the region from the region adjacent to the chorionic plate to a 30% of the whole thickness of region from adjacent to the chorionic plate to the end of the villi, and includes a villi basal portion, and exhibits homogeneous growth characteristics and excellent proliferation characteristics and has remarkably excellent differentiation ability into cartilage, bone and fat compared to other placental tissue-derived stem cells so that it can be effectively used for various tissue regeneration treatments that require regeneration of cartilage, bone and fat, in particular, cartilage regeneration and osteoarthritis treatment.

### [Description of Drawings]

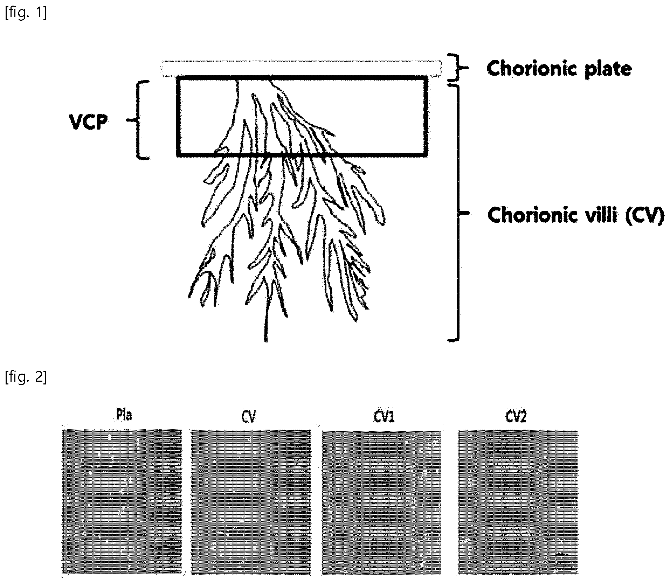

FIG. 1 is a view schematically showing the chorionic plate, the whole villi (CV) and villi adjacent to chorionic plate (VCP).
FIG. 2 is a view showing the result of observing, under a microscope, the morphology of cells obtained by culturing stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2).

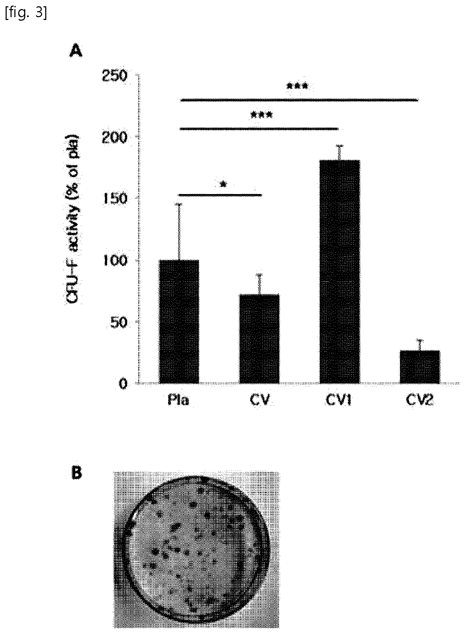

FIG. 3A is a view showing the result of confirming the colony formation capacity of stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2), and FIG. 3B is a view showing the colony size of CV1.

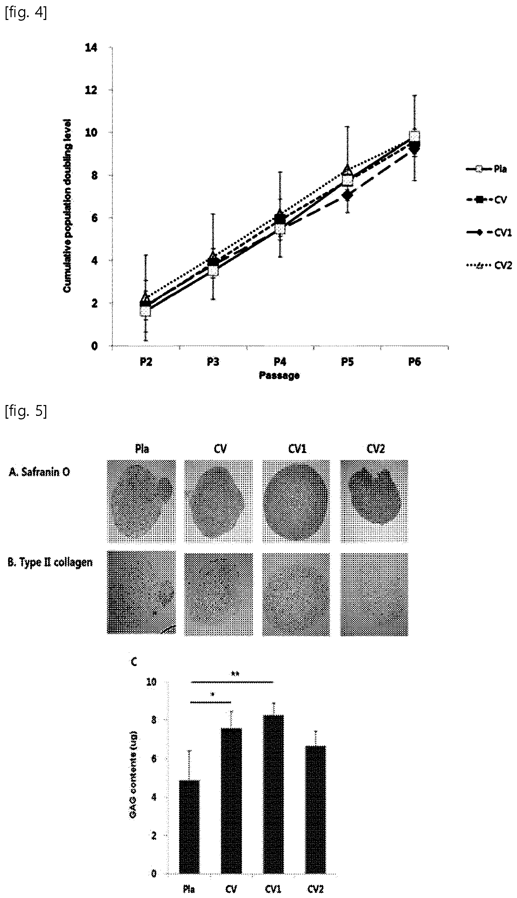

FIG. 4 is a view showing the result of comparing the population doubling time of stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2) according to passage P2 to P6.
FIG. 5 is a view showing the result of confirming the chondrocyte differentiation ability of stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2), FIG. 5A is a view showing the results of safranin O staining, FIG. 5B is a view showing the results of type II collagen staining, and FIG. 5C is a view showing the measurement result of the GAG content.

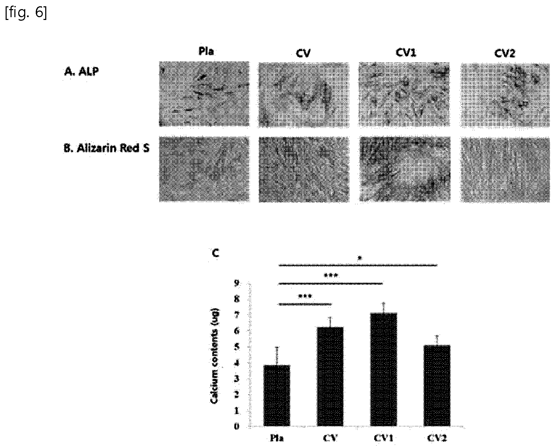

FIG. 6 is a view showing the result of confirming the osteocyte differentiation ability of stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2), FIG. 6A is a view showing the results of ALP staining, FIG. 6B is a view showing the results of alizarin red S staining, and FIG. 6C is a view showing the measurement result of the calcium content.

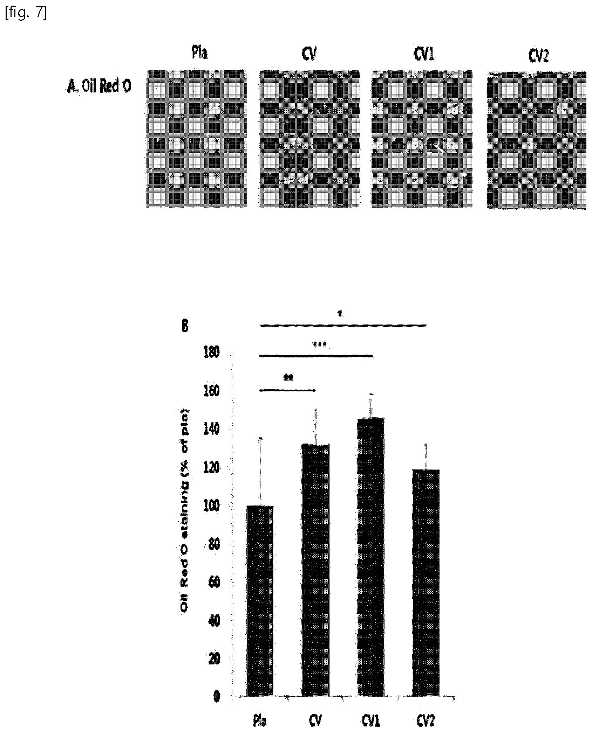

FIG. 7 is a view showing the result of confirming the adipocyte differentiation ability of stem cells derived from whole placenta (Pla), stem cells derived from whole villi (CV), stem cells derived from villi adjacent to chorionic plate (VCP) (CV1), and stem cells derived from portion excluding the villi adjacent to the chorionic plate from whole villi (CV-VCP) (CV2), FIG. 7A is a view showing the results of oil red O staining, and FIG. 7B is a view showing the quantified Oil red O staining results.

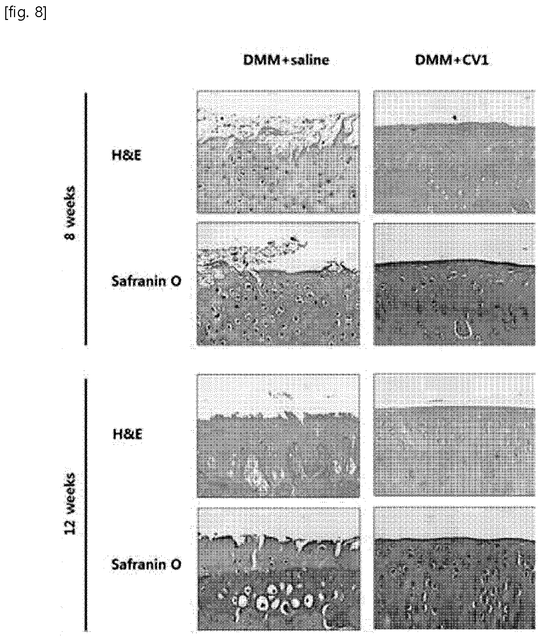

FIG. 8 is a view showing the result of confirming the degree of cartilage regeneration after administrating CV1 to an animal model of cartilage damage.

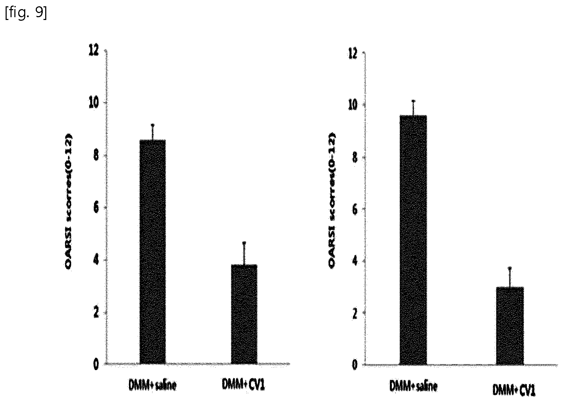

FIG. 9 is a view showing the results of confirming the cartilage protection and regeneration effect in arthritis by measuring the OARSI score after administering CV1 to an animal model of cartilage damage.

### [Best Mode]

The present invention provides stem cells derived from villi adjacent to chorionic plate (VCP) tissue, a cellular therapeutic agent for tissue regeneration including the same, and a composition for tissue regeneration including the same.

Hereinafter, the present invention will be described in detail.

As used herein, the term "stem cell" refers to a cell having the ability to differentiate into two or more different types of cells while having the ability to self-replicate. Stem cells may be classified into totipotent stem cells, pluripotent stem cells, and multipotent stem cells according to their differentiation ability.

As used herein, the term "placenta" refers to an *in vivo* tissue made for a fetus during pregnancy and is in the form of a disc with a weight of 500 g to 600 g, a diameter of 15 cm to 20 cm and a thickness of about 2 cm to 3 cm. One side of the placenta is in contact with the mother and the other side is in contact with the fetus, and nutrients and oxygen are transferred between the blood vessels of the mother and the fetus. The placenta can be generally divided into three layers of the amniotic membrane, the chorion, and the decidua, and in more detail, it can be divided into the amniotic epithelium, the amniotic membrane, the chorion, the trophoblast, and the decidua.

As used herein, the term "villi adjacent to chorionic plate (VCP)" may be a region including the villi basal portion and a portion from the region adjacent to the chorionic plate to a 1/3 point of the whole thickness of the region from adjacent to the chorionic plate to the end of the villi. More specifically, it can be a continuous site starting from the side adjacent to the chorionic plate, corresponding to a portion from the region adjacent to the chorionic plate of the whole villi to the 1/10, 1/9, 1/8, 1/7, 1/6, 1/5, 1/4, and 1/3 point of the distance from the region adjacent to the chorionic plate to the end of the villi. Preferably, it can be a portion from the region adjacent to the chorionic plate to the 1/5 point to 1/3 point of the distance from the region adjacent to the chorionic plate to the end of the villi, that is, a continuous region of up to 20% to 33% of the whole length of the villi with the site adjacent to the chorionic plate of whole villi as the starting point, for example, up to 20%, 21%, 22%, 23%, 24%, 25%, 26%, 27%, 28%, 29%, or 30% of the whole length of villi with the site adjacent to the chorionic plate as the starting point. The villi adjacent to the chorionic plate of the present invention is shown schematically in FIG. 1.

The present invention provides stem cells derived from the villi adjacent to chorionic plate (VCP) tissue, wherein the villi adjacent to the chorionic plate is the villi of the region from the region adjacent to the chorionic plate to a 1/3 point of the whole thickness of the region from adjacent to the chorionic plate to the end of the villi, and includes the basal portion of the villi.

The stem cells derived from villi adjacent to chorionic plate (VCP) tissue of the present invention have a negative surface factor expression characteristic for CD273, CD275 and/or CD321. However, the characteristic of the present invention is a unique characteristic other than the stem cells derived from whole placenta (Pla), the stem cells derived from whole villi (CV) and stem cells derived from portion excluding the villi adjacent to the chorionic plate from whole villi (CV-VCP) defined by the present invention having a positive surface factor expression characteristic for CD273, CD275 and CD321. This may indicate that the stem cells of the present invention are stem cells that are distinguished from other stem cells.

Further, the stem cells of the present invention may be characterized as having positive surface factor expression characteristics for CD49e, CD140b and/or CD142.

The stem cells derived from the villi adjacent to chorionic plate (VCP) tissue of the present invention may be characterized as having surface factor marker expression characteristics of synthetically i) positive for CD44, CD73, CD90 and CD105; ii) positive for CD49e, CD140b or CD142; iii) negative for CD31, CD34, CD45 and HLA-DR; and iv) negative for CD273, CD275 or CD321. For example, while showing positive expression of surface factor marker for CD44, CD73, CD90, and CD105, negative for CD31, CD34, CD45 and HLA-DR, they may be positive for at least one selected from the group consisting of CD49e, CD140b and CD142 and may be negative for at least one selected from the group consisting of CD273, CD275 and CD321. As an example, it may be characterized in that it exhibits surface factor marker expression characteristics that are all positive for CD49e, CD140b and CD142, and all negative for CD273, CD275 and CD321.

Further, the stem cells of the present invention may be characterized as stem cells of fetal cell origin. The placenta is a unique tissue that shows the characteristics derived from the fetus and the mother and consists of tissues showing individual characteristics, including the basement membrane attached to the uterus, the villous tissue that mediates the exchange of substances between the mother and fetal blood as the central tissue of the placenta, and chorion that surrounds the fetus and the amniotic membrane while covering the placenta inside of the placenta. Therefore, even for placental-derived stem cells, the origin of the cells may vary differently depending on the collecting location. It is known that trophoblast-derived stem cells are stem cells originating from maternal cells, villi-derived stem cells are stem cells originating from fetal cells, and whole placental-derived stem cells are stem cells originating from both the fetus and the mother. As a result of chromosomal analysis, it was confirmed that the stem cells derived from the villous tissue adjacent to the chorionic plate tissue of the present invention have a fetal-derived cell chromosomal type. Stem cells derived from villi of the rest excluding the villi adjacent to the chorionic plate (CV-VCP) show the maternal-derived chromosome type. Such results can show that even the same villi-derived stem cells are cells with different cell origins and different characteristics depending on the location of the detailed collecting site.

Further, the stem cells of the present invention may be stem cells obtained from steps of: 1) obtaining villi adjacent to chorionic plate (VCP) by cutting a portion from the region adjacent to the chorionic plate to a 1/3 point of the whole thickness of the region from adjacent to the chorionic plate to the end of the villi; and 2) obtaining a stem cell from the villi adjacent to the chorionic plate.

Further, the present invention relates to a method for separating and obtaining a stem cell derived from villi adjacent to chorionic plate (VCP), the method including: 1) obtaining villi adjacent to chorionic plate (VCP) from a region adjacent to the chorionic plate to a 1/3 point of the whole thickness of the region from adjacent to the chorionic plate to the end of the villi; and 2) obtaining a stem cell from the villi adjacent to the chorionic plate.

Also, in the present invention, the method for separating and obtaining may be used in the same meaning as the method for preparing stem cells derived from villi adjacent to chorionic plate (VCP). The present invention may provide a method for preparing a stem cell derived from villi adjacent to chorionic plate (VCP), the method including: 1) obtaining villi adjacent to chorionic plate (VCP) tissue from the region adjacent to the chorionic plate to a 1/3 point of the whole thickness of the region from adjacent to the chorionic plate to the end of the villi; and 2) obtaining a stem cell from the villi adjacent to the chorionic plate.

Step 1) of the above method is a step of obtaining villi adjacent to the chorionic plate from whole villi, and a method known in the art may be used without limitation to obtain villi adjacent to the chorionic plate. In this case, sterile forceps and a scalpel may be used to obtain the villi adjacent to the chorionic plate by cutting a portion from the region adjacent to the chorionic plate to a 1/3 point of the whole thickness of the region from adjacent to the chorionic plate to the end of the villi. More specifically, a continuous site starting from the side adjacent to the chorionic plate may be obtained by cutting a site corresponding to a portion from the region adjacent to the chorionic plate of the whole villi to the 1/10, 1/9, 1/8, 1/7, 1/6, 1/5, 1/4, and 1/3 point of the distance from the region adjacent to the chorionic plate to the end of the villi. Preferably, it may be obtained by cutting a portion from the region adjacent to the chorionic plate to the 1/5 point to 1/3 point of the distance from the region adjacent to the chorionic plate to the end of the villi, that is, a continuous region of 20% to 33% of the whole length of the villi with the site adjacent to the chorionic plate of whole villi as the starting point. This cutting is performed to divide the while villi into villi adjacent to chorionic plate (VCP) and the CV-VCP region, which is the remaining villi except for the villi adjacent to the chorionic plate from the whole villi. The villi adjacent to the chorionic plate includes the villi basal portion, and the CV-VCP region includes the distal end of the villi.

Step 2) of the above method is a step of obtaining stem cells from the villi adjacent to chorionic plate (VCP) obtained in step 1) in which an enzyme solution is added to the villous adjacent to the chorionic plate tissue to perform an enzymatic reaction, the obtained cells are cultured in a medium supplemented with fetal bovine serum and antibiotics without using a growth factor, and the cells are recovered. The enzymes include trypsin, collagenase, dispase, DNase, RNase, protease, lipase, hyaluronidase, elastase, and the like, but are not limited thereto. The collagenase includes collagenase A, I, II, III or IV and the like.

According to the method of the present invention, stem cells may be specifically isolated from the villi adjacent to the chorionic plate region among villi, and the stem cells isolated in this way differ from whole villi, CV-VCP villi, and placental-derived stem cells and may be characterized as having negative surface factor expression characteristics for CD273, CD275 and CD321. Stem cells obtained through the method of the present invention may exhibit fibroblast-like morphological characteristics, specifically, maintain only single cells, and have excellent colony formation capacity and excellent proliferative ability compared to other tissue-derived stem cells.

Stem cells derived from the villi adjacent to the chorionic plate region according to the present invention can be differentiated into different types of cells, for example, adipocytes, chondrocytes, osteocytes, nerve cells, ligament cells, or tenocytes, but not limited thereto.

As used herein, the "differentiation" generally refers to a phenomenon in which a relatively simple limit is divided into two or more qualitatively different parts and particularly, means a phenomenon in which different structures or functions are specified while the cells are divided, proliferated, and grown, that is, forms or functions are changed so that cells, tissues, and the like of the organism perform given tasks. On the other hand, the "non-differentiation" means a state in which the aforementioned differentiation does not occur and features as the stem cells are yet included.

A method of differentiating the stem cells may be performed according to an existing known method and is not particularly limited thereto. For example, preferably, the method may be a method of differentiating the stem cells into the adipocyte by culturing the stem cells in a medium including dexamethasone, indomethacin, insulin, and 3-isobutyl-1-methylxanthine (IBMX); a method of differentiating the stem cells into the chondrocyte by culturing the stem cells in a medium including dexamethasone, bone morphogenetic protein 6 (BMP-6), transforming growth factor beta (TGF-β), ascorbic acid, and L-proline; a method of differentiating the stem cells into the osteocyte by culturing the stem cells in a medium including dexamethasone, ascorbic acid, β-glycerophosphate, and ascorbic acid-2-phosphate; and the like.

As a method of measuring the degree of differentiation of the stem cells derived from the villi adjacent to chorionic plate region differentiated by the above-described method, a flow cytometry method, an immunocytochemical method, a method of measuring a cell surface marker or a change in form by using a PCR or a gene-expression profile, a method of examining a morphologic change of the cells by using an optical microscope or a confocal microscope, a method of measuring a change in a gene-expression profile, and the like, which are known in the related art, may be used, but is not limited thereto. Preferably, RT-PCR, an oil-red O staining method, a safranin O staining method, a Type II collagen immunohistochemical staining method, an alkaline phosphate (ALP) staining method, an alizarin red S staining method, or the like may be used.

Further, the present invention provides a cellular therapeutic agent or composition for tissue regeneration including, as an active ingredient, a stem cell derived from villi adjacent to chorionic plate (VCP) tissue, in which the villi adjacent to chorionic plate is the villi of the region from the region adjacent to the chorionic plate to a 1/3 point of the whole thickness of the region from adjacent to the chorionic plate to the end of the villi.

Further, the present invention provides a method for tissue regeneration, the method including a step of: treating an individual in need of stem cells, in which the stem cells are derived from villi adjacent to chorionic plate tissue, in which the villi adjacent to chorionic plate is the villi of the region from the region adjacent to the chorionic plate to a 1/3 point of the whole thickness of the region from adjacent to the chorionic plate to the end of the villi and includes the basal portion of the villi.

Further, the present invention provides a method for tissue generation, the method including steps of: 1) obtaining villi adjacent to chorionic plate (VCP) by cutting a portion from the region adjacent to the chorionic plate to a 1/3 point of the whole thickness of the region from adjacent to the chorionic plate to the end of the villi; 2) obtaining stem cells from the villi adjacent to the chorionic plate; and 3) treating an individual in need of the obtained stem cell derived from villi adjacent to chorionic plate.

In the present invention, the treatment includes all of the actions such as administration and injection, and preferably may refer to the action of directly injecting into a site requiring tissue regeneration of an individual in need of treatment.

The stem cells derived from the villi adjacent to chorionic plate (VCP) tissue of the present invention have excellent colony formation capacity and excellent proliferation ability and can differentiate into various types of cells such as adipocytes, chondrocytes, osteocytes, nerve cells, ligament cells or tenocytes. Thus, it can be used as a cellular therapeutic agent and a composition for tissue regeneration for the purpose of tissue regeneration.

As used herein, the term "cellular therapeutic agent" as a drug (U.S. FDA regulations) used for treating, diagnosing, and preventing by using cells and tissues prepared through isolation from the human, culture, and a specific manipulation, means a drug used for treating, diagnosing, and preventing of diseases by using the cells through a series of actions such as *in vitro* proliferating and screening living self, homogeneous, or heterogeneous cells for restoring functions of cells or tissues, changing a biological characteristic of the cells by another method, and the like.

The stem cell derived from villi adjacent to chorionic plate (VCP) tissue according to the present invention may be used in various kinds of treatment protocols which are controlled, reinforced, treated, or replaced by engrafting, transplanting, or infusing a desired cell colony of tissues or organs of the body, for example, a colony of the stem cells or the differentiated cells. The stem cell derived from villi adjacent to chorionic plate (VCP) tissue of the present invention may become a new or changed tissue or be bonded with a biological tissue or structure by replacing or reinforcing an existing tissue.

Preferably, the cellular therapeutic agent of the present invention may be used for regenerating one or more tissues selected from the group consisting of fat, cartilage, bone, nerve, ligament and tendon. In addition, in the present invention, the cartilage may include, without limitation, hyaline cartilage, fibrocartilage or elastic cartilage, and the cartilage is selected from the group consisting of articular cartilage, ear cartilage, nasal cartilage, elbow cartilage, meniscus, knee cartilage, costal cartilage, ankle cartilage, ductal cartilage, occipital cartilage and vertebral cartilage.

Therefore, the cellular therapeutic agent of the present invention may be used for treating bone defect, tendon-ligament defect, adipose tissue defect, cartilage necrosis, osteochondritis, cartilage rupture, cartilage trauma, arthritis, cartilage deficiency or congenital organ softening.

Further, the cellular therapeutic agent or the composition for tissue regeneration of the present invention may be administered directly into a joint or joint cavity, and in this case, it may be administered in the form of an injection. Since the stem cells derived from villi adjacent to chorionic plate (VCP) tissue of the present invention are stem cells with excellent differentiation ability that can be differentiated into cartilage, bone, fat, etc., it can treat or prevent various lesions, such as lesions of articular cartilage by administering to the joint, cartilage, tendon or ligament site. In particular, when the stem cells derived from the villi adjacent to chorionic plate (VCP) tissue of the present invention are administered intra-articularly, the cartilage surface may be regenerated in a soft state with almost no damage, and it may protect and regenerate cartilage so that arthritis does not progress.

In particular, the cellular therapeutic agent or composition for tissue regeneration of the present invention may be formulated in the form of an injection for direct administration of stem cells derived from villi adjacent to chorionic plate (VCP) tissue. In this case, it may further include a hydrogel to be suitable for injection.

The hydrogel that can be used for the cellular therapeutic agent of the present invention may include, without limitation, a hydrogel suitable for injection, known in the art and may include at least one selected from the group consisting of small intestine submucosal tissue, hyaluronic acid, carboxymethyl cellulose (CMC), alginate, chitosan, polyacrylamide, poly(N-isopropylacrylamide), β-glycerophosphate, Pluronic (poly(ethylene oxide)poly(propylene oxide)poly(ethylene oxide)), fibrin, polyethylene oxide (PEO) and a mixture of carboxymethyl cellulose (CMC) and polyethyleneimine (PEI), and in the preferred example of the present invention, hyaluronic acid and pluronic were mixed and used with stem cells derived from the villi adjacent to chorionic plate (VCP) tissue.

The preferred dosage of the cellular therapeutic agent of the present invention varies depending on the condition and weight of the individual, the degree of disease, the drug form, the route and duration of administration, but may be appropriately selected by those skilled in the art. The cellular therapeutic agent is administrated once a day or may be administrated in several divided doses, and the dosage is not intended to limit the scope of the present invention in any way.

Further, the present invention provides the use for the preparation of a therapeutic agent for tissue regeneration by stem cells derived from villous adjacent to the chorionic plate tissue, in which the villi adjacent to chorionic plate is the villi of the region from the region adjacent to the chorionic plate to a 1/3 point of the whole thickness of the region from adjacent to the chorionic plate to the end of the villi, and includes the basal portion of the villi.

Further, the present invention provides the use for tissue regeneration by stem cells derived from villous adjacent to the chorionic plate tissue for tissue regeneration in which the villi adjacent to the chorionic plate is the villi of the region from the region adjacent to the chorionic plate to a 1/3 point of the whole thickness of the region from adjacent to the chorionic plate to the end of the villi and includes the basal portion of the villi.

Since the stem cells derived from villi adjacent to chorionic plate (VCP) tissue are stem cells with excellent differentiation ability that can be differentiated into cartilage, bone, fat, etc., it may treat or prevent various lesions, such as lesions of articular cartilage by administering to the joint, cartilage, tendon or ligament site. In particular, when the stem cells derived from the villi adjacent to chorionic plate (VCP) tissue of the present invention are administered intra-articularly, the cartilage surface may be regenerated in a soft state with almost no damage, and it may protect and regenerate cartilage so that arthritis does not progress. Therefore, the tissue regeneration therapeutic agent may be for treatment of bone defect, tendon-ligament defect, adipose tissue defect, cartilage necrosis, osteochondritis, cartilage rupture, cartilage trauma, arthritis, cartilage deficiency or congenital organ softening, and the use of tissue regeneration may be for cartilage generation.

Further, the present invention provides the cellular therapeutic agent for tissue generation including the stem cell derived from villi adjacent to chorionic plate (VCP) obtained by a method for isolating and obtaining or a method for preparing stem cells derived from villi adjacent to chorionic plate (VCP) including steps of: 1) obtaining villi adjacent to chorionic plate (VCP) by cutting a portion from the region adjacent to the chorionic plate to a 1/3 point of the whole thickness of the region from adjacent to the chorionic plate to the end of the villi; and 2) obtaining a stem cell from the villi adjacent to the chorionic plate; or the composition for tissue generation including the stem cell derived from villi adjacent to chorionic plate (VCP) obtained by a method for isolating and obtaining or a method for preparing stem cells derived from villi adjacent to chorionic plate (VCP) including steps of: 1) obtaining villi adjacent to chorionic plate (VCP) by cutting a portion from the region adjacent to the chorionic plate to a 1/3 point of the whole thickness of the region from adjacent to the chorionic plate to the end of the villi; and 2) obtaining a stem cell from the villi adjacent to the chorionic plate

Further, the present invention provides a method for tissue generation, the method including steps of: 1) obtaining villi adjacent to chorionic plate (VCP) by cutting a portion from the region adjacent to the chorionic plate to a 1/3 point of the whole thickness of the region from adjacent to the chorionic plate to the end of the villi; 2) obtaining a stem cell from the villi adjacent to the chorionic plate; and 3) treating an individual in need of the obtained stem cell derived from villi adjacent to chorionic plate.

The stem cells derived from villi adjacent to chorionic plate (VCP) tissue according to the present invention have excellent proliferation and differentiation abilities and thus have excellent tissue regeneration effects. Contents that overlap with those described in the cellular therapeutic agent for tissue regeneration are excluded in order to avoid the complexity of the description.

Hereinafter, the present invention is described in more detail through the following examples. These examples are for explaining the present invention in detail, but the scope of the present invention is not limited by these examples.

### [Modes of the Invention]

### Example 1: Isolation of villi adjacent to chorionic plate of placenta and obtainment of stem cells

The amniotic membrane was removed from the placental tissue, and the decidua was removed using sterile scissors to obtain the entire trophoblast layer (chorion) with a size of about 5 × 5 × 3 cm. It was transferred to a 150 mm dish and washed 5 or more times using PBS to remove blood and blood cells. In order to separate only the villi, the entire trophoblast was separated again into villi and intervillous, and the separated villi were washed 5 or more times using PBS to remove the remaining blood and blood cells. The villi tissue only corresponding to the region adjacent to chorionic plate corresponding to the length of about 20% of full-length of the washed villi from the chorionic plate (from the region adjacent to the chorionic plate of the whole villi to the 1/5 point of the distance from the region adjacent to the chorionic plate to the end of the villi) was cut with a sterilized forceps and scalpel to obtain the villi portion. In order to distinguish the obtained cut villi tissue including the basal portion of the villi from whole villi (chorionic villi, CV), its portion is defined as villi adjacent to chorionic plate (VCP). The villi adjacent to the chorionic plate are schematically shown in FIG. 1.

The villi adjacent to the chorionic plate (hereinafter, VCP) was transferred to a 50 ml tube, added with an a-MEM medium including 0.2% collagenase, and then reacted for about 2 hours by using an agitator at 37°C to obtain cells derived from VCP. The obtained cells derived from VCP were filtered with a mesh of 100 µm to remove a non-decomposed tissue, added with an a-MEM medium including fetal bovine serum and antibiotics and then centrifuged for 4 minutes at 25°C and 1500 rpm. The supernatant was removed, and then the remaining precipitated cells were added with an a-MEM medium including fetal bovine serum and antibiotics without a growth factor and cultured under a condition of 37°C and 5% CO₂. The stem cells derived from VCP were obtained by screening the cells attached to the bottom of the culture container from the culture. The obtained stem cells were referred to as "CV1"

### Comparative Example 1: Obtaining stem cells derived from other tissues

### 1-1. Obtaining stem cells derived from whole placenta

The whole placental tissue was cut into about 5 × 5 × 3 cm and washed with PBS to remove blood and blood cells from the placental tissue. The washed placental tissue was added with an a-MEM medium including 0.2% collagenase. Placental cells were obtained by reaction using an agitator at 37°C. The obtained placental cells were filtered with a mesh of 100 µm to remove a non-decomposed tissue, added with an a-MEM medium including fetal bovine serum and antibiotics and then centrifuged for 4 min at 25°C and 1000 rpm. The supernatant was removed, and then the remaining precipitated cells were added with an a-MEM medium including fetal bovine serum and antibiotics without a growth factor and cultured under a condition of 37°C and 5% CO₂. The stem cells derived from whole placenta (Pla) were obtained by screening the cells attached to the bottom of the culture container from the culture.

### 1-2. Obtaining stem cells derived from partial placental tissue

The amniotic membrane was removed from the placental tissue, and the decidua was removed to obtain the entire trophoblast layer with a size of about 5 × 5 × 3 cm. It was transferred to a 150 mm dish and washed 5 or more times using PBS to remove blood and blood cells. In order to separate only the villi, the entire trophoblast was separated again into villi and intervillous, and the separated villi were washed 5 or more times using PBS to remove the remaining blood and blood cells. The remaining tissue parts except for the villi adjacent to chorionic plate (VCP) described in Example 1 in the whole villi (chorionic villi, CV) were defined as CV-VCP, meaning the portion excluding the villi adjacent to chorionic plate from whole villi. Whole villi or CV-VCP tissue was transferred to a 50 ml tube, added with an a-MEM medium including 0.2% collagenase, and then reacted for about 2 hours by using an agitator at 37°C to obtain cells derived from each tissue. The obtained cells were filtered with a mesh of 100 µm to remove a non-decomposed tissue, added with an a-MEM medium including fetal bovine serum and antibiotics and then centrifuged for 4 minutes at 25°C and 1500 rpm. The supernatant was removed, and then the remaining precipitated cells were added with an a-MEM medium including fetal bovine serum and antibiotics without a growth factor and cultured under a condition of 37°C and 5% CO₂. The stem cells were obtained by screening the cells attached to the bottom of the culture container from the culture. The stem cells derived from the whole villi of the trophoblast layer were referred to as "CV," and stem cells derived from CV-VCP were referred to as "CV2."

### Example 2: Culture and cell morphology of stem cells derived from villi adjacent to chorionic plate (CV1)

The CV1 stem cells obtained in Example 1 were washed with PBS and then cultured by replacing a DMEM medium including fetal bovine serum and antibiotics without a growth factor every 2 to 3 days. When the stem cells were grown 80% or more, the stem cells were treated with TryPLE to be isolated from the culture container, and the isolated stem cells were diluted in a ratio of 1/5 and then cultured in another culture container to perform a subculture. Cell morphology after culture was observed under a microscope. In addition, using the whole placenta (Pla) and other partial tissue-derived stem cells obtained in Comparative Example 1, subculture was performed in the same manner, and the cell morphology after culture was observed under a microscope. The results are shown in each FIG. 2.

As shown in FIG. 2, it was confirmed that the CV1 stem cells according to the present invention exhibited morphological characteristics of a fibroblast shape and specifically maintained only single cells. On the other hand, whole placenta (Pla) and other partial tissue-derived stem cells showed morphological characteristics of a fibroblast shape, but a number of different types of cells were mixed.

That is, compared to CV1 stem cells according to the present invention, it was confirmed that in CV1 stem cells before and after subculture, only single cells were specifically maintained, but the cells having different shapes were mixed in stem cells derived from the whole placenta and other partial tissues.

### Example 3: Analysis of colony formation capacity of stem cells derived from villi adjacent to chorionic plate (CV1)

A colony formation capacity of the CV1 stem cells obtained in Example 1 was verified. More specifically, the first subculture of the CV1 stem cells obtained in Example 1 was performed by the method of Example 2, and the stem cells were seeded by 5 × 10³ in a dish of 100 mm at the time when the subculture was completed and then cultured in an a-MEM medium supplemented with fetal bovine serum and antibiotics without a growth factor for 14 days. Thereafter, the number of colonies formed in each stem cell was counted. Further, by using the stem cells derived from the whole placenta and other partial placenta tissues obtained in Comparative Example 1, the population doubling time and the colony formation capacity were measured by the same method. In the case of the colony formation capacity, the result value of the stem cells derived from the whole placenta was converted to 100%. The results are illustrated in FIGS. 3 to 4.

As shown in FIG. 3A, it was confirmed that stem cells derived from VCP (CV1) showed a significantly excellent colony formation capacity as compared colony formation capacity compared to whole placenta (Pla), whole villi-derived (CV), and stem cells derived from CV-VCP excluding the villi adjacent to the chorionic plate from whole villi (CV2). As shown in FIG. 3B, it was confirmed that the colony size of stem cells derived from VCP (CV1) was formed to be 3 to 4 mm or more.

The results confirmed that CV1 stem cells showed a colony formation capacity more than twice that of CV2 stem cells so that these were stem cells with very excellent colony formation capacity compared to stem cells derived from whole villi and other villi portions.

Further, as shown in FIG. 4, it was confirmed that the population doubling time was maintained similarly from P2 to P4, but after P5, the CV1 stem cells of the present invention exhibited the lowest population doubling time. Through these results, it was confirmed that the stem cells derived from the villi adjacent to the chorionic plate (CV1) had excellent proliferative capacity.

### Example 4: Analysis of surface marker of stem cells derived from villi adjacent to chorionic plate (CV1)

In order to verify immunological properties of CV1 obtained in Example 1, surface markers were analyzed using 242 Human Cell Surface Marker Screening Panels. First, the villi adjacent to the chorionic plate was obtained according to Example 1 and was washed with PBS. This was treated with TryPLE to collect the stem cells, and the stem cells were centrifuged for 5 min at 1200 rpm. After the supernatant was removed and the stem cells were washed with PBS, the stem cells were centrifuged for 5 min at 1200 rpm. After the supernatant was removed and the stem cells were suspended in PBS and were passed through 40 µm cell strainer to prepare and mix BD Pharmingen Stain Buffer + EDTA. 100 µl of the mixture was dispensed into each well (100,000 pieces/well) of Falcon^{®} round bottom 96-well plate (Cat. 353910). After that, 20 µl of the corresponding antibody was dispensed into each well. After incubation at 4°C for 30 minutes, BD Pharmingen Stain Buffer + EDTA was added to each well for washing, followed by centrifugation at 1200 rpm for 5 minutes. The supernatant was again carefully removed, and they were washed with BD Pharmingen Stain Buffer + EDTA, followed by centrifugation at 1200 rpm for 5 minutes. After that, 100 µl of secondary antibody was dispensed into each well. For washing, BD Pharmingen Stain Buffer + EDTA was added to each well, followed by centrifugation at 1200 rpm for 5 minutes. The supernatant was removed, and the washing process was carefully repeated twice using PBS. The supernatant was removed, and 150 µl of BD Pharmingen Stain Buffer + EDTA was dispensed into each well. At least 10,000 cells per well were analyzed for surface markers using a flow cytometer (FACS). In addition, the surface markers of stem cells derived from the whole placenta and other partial placental tissue obtained in Comparative example 1 were analyzed in the same manner. The results are shown in Tables 1 and 2.

**[Table 1]**

| | | | | Unit: % |
|---|---|---|---|---|
| | Pla | CV | CV1 | CV2 |
| CD31 | 0.61 | 0.52 | 0.34 | 0.26 |
| CD34 | 0.89 | 1.24 | 1 | 0.54 |
| CD44 | 96.30 | 95.92 | 97.82 | 80.44 |
| CD4S | 1.25 | 1.32 | 2.24 | 0.84 |
| CD73 | 95.80 | 92.26 | 96.91 | 20.72 |
| CD90 | 96.12 | 97.68 | 93.26 | 79.4 |
| CD105 | 97.89 | 94.62 | 93.62 | 40.16 |
| HLA-DR | 2.45 | 2.64 | 2.24 | 2.87 |

**[Table 2]**

| | | | | Unit: % |
|---|---|---|---|---|
| | Pla | CV | CV1 | CV2 |
| CD49e | 69.2 | 66.32 | 90.7 | 5.6 |
| CD140b | 80.9 | 59.6 | 16.56 | 51.02 |
| CD142 | 72.5 | 52.64 | 87.66 | 31.46 |
| CD273 | 54.2 | 12.8 | 1.44 | 32.18 |
| CD275 | 15.3 | 10.7 | 3.41 | 15.66 |
| CD321 | 48.2 | 34.32 | 1.38 | 31.70 |

If the expression level of the surface marker was less than 5%, it was classified as a negative marker, and if it was 6% or more, it was classified as a positive marker. Positive markers were classified as low when they were 6% or more and less than 30%, middle when they were 30 to 70%, and high when they were 70% or more. According to their expression levels, Tables 3 and 4 show marker expression.

**[Table 3]**

| | Pla | CV | CV1 | CV2 |
|---|---|---|---|---|
| CD31 | neg | neg | neg | neg |
| CD34 | neg | neg | neg | neg |
| CD44 | high | high | high | high |
| CD45 | neg | neg | neg | neg |
| CD73 | high | high | high | low |
| CD90 | high | high | high | high |
| CD105 | high | high | high | low |
| HLA-DR | neg | neg | neg | neg |

**[Table 4]**

| | Pla | CV | CV1 | CV2 |
|---|---|---|---|---|
| CD49e | mid | mid | high | neg |
| CD140b | high | mid | low | mid |
| CD142 | high | mid | high | mid |
| CD273 | mid | low | neg | mid |
| CD275 | low | low | neg | low |
| CD321 | mid | mid | neg | mid |

As shown in Table 3, it was confirmed that CV1 of the present invention showed negative for CD273, CD275, and CD321 markers, unlike stem cells derived from whole placenta and stem cells derived from villi (CV), and in particular, such expression was different from that of the stem cells derived from villi excluding the villi adjacent to the chorionic plate (CV-VCP) (CV2). The results confirmed that the stem cell was a new stem cell exhibiting completely different CD marker expression characteristics from other comparative stem cells.

In addition, the above results show that stem cells derived from the whole villi are in a state in which stem cells showing various CD marker expression patterns are mixed. As in the present invention, it can be confirmed that when the stem cells are subdivided and separated from the villi adjacent to chorionic plate, more uniform stem cells can be obtained.

### Example 5: Comparison of cell origin of stem cells derived from villi adjacent to chorionic plate (CV1)

An experiment was performed to confirm whether the cell origin of CV1 obtained in Example 1 belongs to different cell groups compared to stem cells derived from whole placenta or villi and stem cells derived from CV-CVP excluding the villi adjacent to the chorionic plate (CV2).

It is known that the placenta is a special organ in which cells derived from both mother and fetus are mixed, and the placenta contains both cells derived from maternal and fetal cells, and in general, the trophoblast is of maternal origin and the villi are of fetal origin. Accordingly, an experiment was performed to confirm the cell origins of CV1 obtained in Example 1 and the cells of Comparative Example 1. The cells obtained from P5, which are known to be widely applied in clinical practice, were plated on a slide and were stained using X and Y chromosome probes labeled with Cy3 (red) and fluorescein isothiocyanate (green), respectively (Vysis, Downers Grove, IL, USA). In slide analysis, after staining the cell nucleus with DAPI, and confirming it using a spectral red and green filter under X1000 magnification, the X and Y chromosomes expressed in the cells were counted to confirm the cell origin, and the results are shown in Table 5.

**[Table 5]**

| | Pla | CV | CV1 | CV2 |
|---|---|---|---|---|
| Cell origin | XX or XY | XY | XY | XX |

As shown in Table 5, it was confirmed that in the case of Pla, XX and XY could be mixed in the initial passage, but 100% XX or 100% XY appeared in P5. Further, it was confirmed that CV and CV1 had XY. On the other hand, it was confirmed that CV2 was originated from 100% XX cells in P5.

That is, as shown in Table 5, CV1 cells showed a fetal-derived cell chromosomal type, not the mother's, whereas CV2 showed a maternal-derived chromosomal type, confirming that they were stem cells having different origins from each other.

### Example 6: Confirmation of ability to differentiate into chondrocyte of stem cells derived from villi adjacent to chorionic plate (CV1)

In order to verify differentiation into chondrocyte of the stem cells derived from villi adjacent to chorionic plate (CV1) obtained in Example 1, the stem cells were cultured for 3 weeks in a known chondrocyte differentiation-induced medium (a DMEM medium including 0.1 µM dexamethasone, 50 µg/ml ascorbic acid, 40 µg/ml L-proline, 10 ng/ml TGF-β3, 500 ng/ml BMP-6, and 50 mg/ml ITS premix) to induce the differentiation into the chondrocytes. In order to measure the degree of differentiation of the stem cells into chondrocytes, a safranin-O staining method and an immunohistochemical staining method using Type II collagen were performed according to the existing known method. Also, after culturing for 3 weeks, the content of sulfated glycosaminoglycan (GAG) was measured using a glycan sulfated GAG assay kit (Biocolor Ltd., Carrickfergus, County Antrim, UK). Absorbance was measured in a 96-well plate at a wavelength of 656 nm using a microplate reader, and the results are shown in FIG. 5.

As shown in FIG. 5, it was confirmed that the CV1 stem cells according to the present invention had superior chondrocyte differentiation ability compared to CV and CV2.

### Example 7: Confirmation of ability to differentiate into osteocyte of stem cells derived from villi adjacent to chorionic plate (CV1)

In order to verify differentiation into osteocyte of the stem cells derived from villi adjacent to chorionic plate (CV1) obtained in Example 1, the stem cells were cultured for 4 weeks in a known osteocyte differentiation-induced medium (a DMEM medium including 10% FBS, 1% anti-biotics, 100 µM dexamethasone, 50 mM of ascorbic acid-2-phosphate, 10 µM β-glycerophosphate, and 250 µM ascorbic acid) to induce the differentiation into the osteocyte. In this case, at the time when two weeks elapsed after the differentiation induction started, an alkaline phosphate (ALP) staining method was performed according to the existing known method, and at the time when four weeks elapsed, an alizarin red S staining method was performed according to the existing known method to analyze the degree of the differentiation into osteocyte. Further, in order to confirm the degree of differentiation of the stem cells derived from the whole placenta and other partial placenta tissues obtained in Comparative Example 1 into osteocytes in the same manner, they were treated with 0.6 M HCl and stored at 37°C for 24 hours for measurement of intracellular calcium at the time when four weeks elapsed. Then, absorbance was measured at 565 nm using the o-cresolphthalein complexone method (Pointe Scientific, Canton, MI, USA), and the calcium concentration was normalized and quantified. The results are shown in FIG. 6.

As shown in FIG. 6, it was confirmed that the stem cells derived from villi adjacent to chorionic plate (CV1) according to the present invention had superior osteocyte differentiation ability compared to CV and CV2.

### Example 8: Confirmation of ability to differentiate into adipocyte of stem cells derived from villi adjacent to chorionic plate (CV1)

In order to verify differentiation into adipocyte of the stem cells derived from villi adjacent to chorionic plate (CV1) obtained in Example 1, the stem cells were alternately added with the known adipocyte differentiation-induced medium I (a DMEM medium including 10% FBS, 1% antibiotics, 1 µM dexamethasone, 20 µM indomethacin, 10 µM insulin, and 50 µM 3-isobutyl-1-methylxanthine (IBMX)) and the adipocyte differentiation-induced medium II (a DMEM medium including 10% FBS, 1% anti-biotics, and 10 µM insulin) was alternatively added for 3 to 4 days and cultured for 3 weeks to induce differentiation into adipocytes. In order to measure the degree of the differentiation of the stem cells into the adipocyte, oil red O staining was performed according to the existing known method. Further, for measurement of the differentiation of the stem cells derived from the whole placenta, other partial placenta tissues, and other tissues obtained in Comparative Example 1 into adipocytes in the same manner, the cells were treated with the 100% isopropanol solution to dissolve the lipid, measuring the absorbance at 500 nm. The results are shown in FIG. 7.

As shown in FIG. 7, it was confirmed that the stem cells derived from villi adjacent to chorionic plate (CV1) according to the present invention had superior adipocyte differentiation ability compared to CV and CV2.

### Example 9: Verification of effect of stem cells derived from villi adjacent to chorionic plate (CV1) as cell therapeutic agent in animal model of arthritis

In order to verify the effect of stem cells derived from the villi adjacent to the chorionic plate (CV1) obtained in Example 1 as a cell therapeutic agent in an animal model of cartilage damage, the following experiment was performed. More specifically, in order to produce an arthritic animal model in wistar rats (rat, male, 12 weeks old), a healthy rat was selected and anesthetized with appropriate amounts of ketamine and rompun according to body weight. It was verified that the rat was sufficiently general-anesthetized. After the knee joints of both pelvic limbs were shaved and fixed with a plaster while the posture was maintained. Both knee joints were disinfected with povidone, the kneecap was detected and the location was verified, the inside of the knee joints was reached by a paramedian approach along a cut line through the top and the bottom of the knee joint and the inside of the kneecap, the knee joint was bent while the kneecap was bent back outside, and then the inside of the joint was observed. After it was verified that there was no specific pathological finding, a model of destabilization of the medial meniscus (DMM) was created by damaging medial meniscus. As such, it was verified that after 8 weeks after inducing the damage, using a syringe, 1 ml of CV1 stem cell suspension was mixed with 5% hyaluronic acid and 10% pluronic, and then 20 µl was injected into the joint cavity of the animal model. After confirming that the rats woke up from anesthesia, they were allowed to move freely. Analgesics and antibiotics were administered to prevent infection for 3 days after surgery. After 8 weeks and 12 weeks after administration, sections of the damaged and treated joint areas were obtained from each rat, and H&E staining and safranin O staining were performed. The degree of regeneration of newly formed cartilage was analyzed through quantification using OARSI score. The results are shown in FIGS. 8 and 9, respectively.

As shown in FIG. 8, it was confirmed that 8 and 12 weeks after CV1 stem cells were injected, the cartilage surface was smooth and regenerated with little or no cartilage damage compared to the control group injected with saline.

Further, as shown in FIG. 9, in the OARSI score, the group injected with CV1 stem cells showed a lower score (3.8 ± 0.8) than the control group (8.6 ± 0.5) after 8 weeks, and the group injected with CV1 stem cells showed a lower score (3.0 ± 0.7) than the control group (9.6 ± 0.5) after 16 weeks. Therefore, it was found that CV1 stem cells play a role in protecting and regenerating cartilage so that arthritis does not progress anymore during the course of arthritis.

Through the above experimental results, it was confirmed that the conventional stem cells derived from the whole placenta are mixed with stem cells derived from partial placental tissues having various characteristics, so the ability to differentiate into different cells is not uniformly shown, whereas stem cells derived from villi adjacent to chorionic plate, which is a partial placental tissue, (CV1) according to the present invention exhibits superior properties compared to conventional stem cells derived from whole placenta in terms of excellent differentiation capacity and homogeneity for various characteristics of other stem cells. In particular, the CV1 stem cells according to the present invention showed consistent patterns in the characteristics of growth, proliferation, morphology and differentiation than stem cells derived from other partial placental tissue and showed the most excellent stem cell characteristics. Therefore, it was confirmed that the CV1 stem cells are used to improve the differentiation efficiency into a target cell and are usefully used as a cellular therapeutic agent in various diseases.

## Claims

1. A stem cell derived from villi adjacent to chorionic plate (VCP) tissue, wherein the villi adjacent to the chorionic plate is the villi of the region from the region adjacent to the chorionic plate to a 1/3 point of the whole thickness of the region from adjacent to the chorionic plate to the end of the villi, and includes the basal portion of the villi.

2. The stem cell of claim 1, wherein the stem cell has a negative surface factor expression characteristic for CD273, CD275 or CD321.

3. The stem cell of claim 1, wherein the stem cell has surface factor marker expression characteristics of
i) positive for CD44, CD73, CD90 and CD105;
ii) positive for CD49e, CD140b or CD142;
iii) negative for CD31, CD34, CD45 and HLA-DR; and
iv) negative for CD273, CD275 or CD321.

4. The stem cell of claim 1, wherein the stem cell is a stem cell of fetal cell origin.

5. The stem cell of claim 1, wherein the stem cell is obtained from steps of:
1) obtaining villi adjacent to chorionic plate (VCP) by cutting a portion from the region adjacent to the chorionic plate to a 1/3 point of the whole thickness of the region from adjacent to the chorionic plate to the end of the villi; and
2) obtaining a stem cell from the villi adjacent to the chorionic plate.

6. A method for separating and obtaining stem cells derived from villi adjacent to chorionic plate (VCP), the method comprising:
1) obtaining villi adjacent to chorionic plate (VCP) by cutting a portion from the region adjacent to the chorionic plate to a 1/3 point of the whole thickness of the region from adjacent to the chorionic plate to the end of the villi; and
2) obtaining stem cells from the villi adjacent to the chorionic plate.

7. The method of claim 6, wherein the stem cell obtained from the villi adjacent to the chorionic plate has a negative surface factor expression characteristic for CD273, CD275 or CD321.

8. A cellular therapeutic agent for tissue regeneration comprising the stem cell of claim 1 as an active ingredient.

9. The cellular therapeutic agent of claim 8, wherein the tissue includes at least one selected from the group consisting of fat, cartilage, bone, nerve, ligament, and tendon.

10. The cellular therapeutic agent of claim 9, wherein the cartilage is hyaline cartilage, fibrocartilage or elastic cartilage.

11. The cellular therapeutic agent of claim 9, wherein the cartilage is selected from the group consisting of articular cartilage, ear cartilage, nasal cartilage, elbow cartilage, meniscus, knee cartilage, costal cartilage, ankle cartilage, ductal cartilage, occipital cartilage and vertebral cartilage.

12. The cellular therapeutic agent of claim 8, wherein the cellular therapeutic agent is for treatment of bone defect, tendon-ligament defect, adipose tissue defect, cartilage necrosis, osteochondritis, cartilage rupture, cartilage trauma, arthritis, cartilage deficiency or congenital organ softening.

13. The cellular therapeutic agent of claim 8, wherein the cellular therapeutic agent is an injection.

14. The cellular therapeutic agent of claim 8, wherein the cellular therapeutic agent further includes a hydrogel.

15. The cellular therapeutic agent of claim 14, wherein the hydrogel includes at least one selected from the group consisting of small intestine submucosal tissue, hyaluronic acid, carboxymethyl cellulose (CMC), alginate, chitosan, polyacrylamide, poly(N-isopropylacrylamide), β-glycerophosphate, pluronic(poly(ethylene oxide)poly(propylene oxide)poly(ethylene oxide)), fibrin, polyethylene oxide and a mixture of carboxymethyl cellulose (CMC) and polyethyleneimine (PEI).

16. A composition for tissue regeneration comprising the stem cell of claim 1 as an active ingredient.

17. A method for tissue regeneration, the method comprising a step of:
treating an individual in need of stem cells,
wherein the stem cells are derived from the villi adjacent to chorionic plate tissue, in which the villi adjacent to chorionic plate is the villi of the region from the region adjacent to the chorionic plate to a 1/3 point of the whole thickness of the region from adjacent to the chorionic plate to the end of the villi, and includes the basal portion of the villi.

18. A method for tissue generation, the method comprising steps of:
1) obtaining villi adjacent to chorionic plate (VCP) by cutting a portion from the region adjacent to the chorionic plate to a 1/3 point of the whole thickness of the region from adjacent to the chorionic plate to the end of the villi;
2) obtaining stem cells from the villi adjacent to the chorionic plate; and
3) treating an individual in need of the obtained stem cells derived from the villi adjacent to chorionic plate.
